# FASCICULE DE BREVET EUROPEEN

(11) **EP 1 213 995 B1**
(45) Date de publication et mention de la délivrance du brevet: **13.08.2008**
(21) Numéro de dépôt: 00962622.7
(22) Date de dépôt: 13.09.2000
(51) Int. Cl.: A61B 6/02, H05G 1/64, G02B 27/02

(54) **DISPOSITIF DE RADIOLOGIE COMPORTANT DES MOYENS D'AGRANDISSEMENT D'IMAGES PERFECTIONNEES**
RADIOLOGISCHE ANORDNUNG MIT VERBESSERTEN BILDVERGRÖSSERUNGSMITTELN
RADIOLOGY DEVICE COMPRISING IMPROVED IMAGE ENLARGING MEANS

(30) Priorité: 14.09.1999 FR 9911469
(43) Date de publication de la demande: 19.06.2002
(73) Titulaire: NANO UV, 91140 Villebon sur Yvette (FR)
(72) Inventeur: CHOI, Peter, F-91400 Orsay (FR)
(74) Mandataire: Pontet, Bernard
(86) Numéro de dépôt international: PCT/FR2000/002524
(87) Numéro de publication internationale: WO 2001/019250

(56) Documents cités:
- EP-A- 0 644 712
- EP-A- 0 858 772
- US-A- 3 912 936
- US-A- 4 467 351
- US-A- 4 896 344
- US-A- 5 949 811

## Description

La présente invention concerne de manière générale les dispositifs de radiologie médicale. Plus précisément, l'invention concerne un dispositif permettant d'obtenir des images numériques de haute résolution d'organes ou de tissus que l'on souhaite examiner (que l'on désignera par souci de simplification dans la suite de ce texte par le terme générique de « sujet »), ainsi que de toute région désirée du sujet.

Les dispositifs de radiologie qui mettent en oeuvre une source de rayons X et un module permettant de visualiser la trace des rayons X ayant traversé le sujet sont connus et largement répandus depuis de nombreuses années. On peut globalement établir la typologie suivante pour ces dispositifs :
- les dispositifs de radiographie, dans lesquels le sujet est intercalé entre une source de rayon X et un film sensible aux rayons X. Ce type de dispositif qui fut historiquement le premier utilisé et qui est le plus répandu, fournit ainsi des images statiques du sujet qui doit rester immobile lors de son exposition aux rayons X pendant un temps suffisant pour obtenir une impression du film par les rayons X. Ce type de dispositif a rendu de grands services ; il comporte néanmoins des inconvénients dont les principaux sont les suivants :
   ➢ limitation à la production d'images statiques, ce qui ne permet pas de visualiser l'évolution dynamique du sujet pour caractériser certains aspects de son fonctionnement,
   ➢ exposition répétée des radiologues à des rayons X et risque sanitaire qui en découle,
- les dispositifs de fluoroscopie offrent quant à eux l'accès à des images dynamiques. Dans ces dispositifs, le sujet est intercalé entre une source de rayons X et des moyens de visualisation qui convertissent en temps réel le rayonnement X en une image visible. Ces moyens peuvent ainsi offrir une visualisation :
   ✔ directe. Dans ce cas, le radiologue visualise directement les images « primaires » qui sont les premières images formées par les moyens de visualisation à partir des rayons X. Les moyens de visualisation consistent alors en un convertisseur du type écran à revêtement de phosphore,
   ✔ ou indirecte. Dans ce cas, le dispositif comprend des moyens pour l'acquisition des images primaires en sortie d'un convertisseur (celui-ci pouvant être du type écran à phosphore), parune chaîne pouvant inclure une caméra vidéo filmant tout le champ d'un écran de sortie du convertisseur pour former une image « secondaire », des moyens de numérisation de l'image et des moyens de traitement, de stockage et de distribution des images à des terminaux différents (pouvant être sur des sites différents). Le document US 4896 344 donne un exemple d'un tel dispositif.

Dans les deux cas (visualisation directe et Indirecte), les moyens de visualisation permettent une vision dynamique de l'évolution temporelle du sujet (visualisation du fonctionnement d'organes en mouvement), ce qui constitue un avantage et offre des possibilités de mise en oeuvre accrues (enregistrement de séquences illustrant le fonctionnement du sujet, assistance opératoire en direct, ...).

Ces dispositifs de fluoroscopie comportent cependant également des inconvénients, parmi lesquels une qualité d'image inférieure à celle des images de radiographie (notamment en termes de contraste), par suite de la nécessaire réduction de l'intensité de l'émission de rayons X pour des raisons de sécurité du radiologue (et des patients), l'exposition au rayonnement étant prolongée.

Pour tenter de diminuer l'importance de ce problème lié aux dispositifs de fluoroscopie, les fabricants ont mis en oeuvre des intensificateurs qui permettent de convertir le rayonnement X en une image optique avec un rendement élevé (c'est-à-dire en produisant un nombre de photons élevé par rayon X Incident).

En augmentant l'intensité des images produites et en améliorant ainsi leur contraste et leur netteté, ces dispositifs permettent d'abaisser l'intensité du rayonnement X à un niveau inférieur à celui mis en oeuvre en radioscopie; ils peuvent fonctionner en mode de visualisation directe, ou indirecte. Dans les deux cas, les intensificateurs comprennent une interface de sortie pour présenter les images primaires à un observateur, ou les transmettre à une chaîne d'acquisition d'images.

Les dispositifs de fluoroscopie constituent ainsi un moyen avantageux de procéder à des examens radiologiques de bonne qualité. Il est par ailleurs possible de procéder à l'examen du sujet selon deux types de procédures :
- uniquement à partir d'images couvrant un champ unique contenant la ou les zone(s) d'intérêt,
- ou bien en effectuant des prises de vues successives de zones d'intérêt différentes.

Le deuxième type de procédure offre l'avantage d'une plus grande souplesse d'utilisation, permettant d'effectuer dans un premier temps une prise de vue en champ large pour identifier des zones d'intérêt, puis de centrer successivement le dispositif sur chacune de ces zones.

Pour ce type d'utilisation, notamment en fluoroscopie indirecte, on prévoit généralement des moyens d'acquisition et d'agrandissement d'image pour recueillir les images primaires dans leur globalité, puis procéder à un agrandissement d'une partie de l'image primaire centrée sur la zone désirée.

Mais ce dernier type d'utilisation présente l'inconvénient de dégrader la résolution des images secondaires qui seront visualisées, étant donné que les images secondaires qui sont agrandies ont préalablement été discrétisées par les moyens d'acquisition : la résolution de l'image observée est dans ce cas N fois inférieure à la résolution des moyens d'acquisition , N étant le coefficient d'agrandissement.

De plus, dans le cas des dispositifs à intensificateur, la résolution de l'image primaire est déjà fortement limitée par la résolution de l'intensificateur lui-même, qui est couramment de l'ordre de 1 à 2 paires de lignes par millimètre seulement.

Un tel inconvénient peut se traduire par l'impossibilité de détecter certains détails de très petite taille, comme par exemple certains symptômes précoces du cancer de l'estomac qui sont de taille millimétrique.

En outre, les intensificateurs des dispositifs de fluoroscopie comprennent généralement des écrans d'entrée courbes qui produisent des aberrations dans certaines parties de l'image.

Un but de l'invention est de pallier aux inconvénients évoqués ci-dessus et de permettre, d'une part de réaliser un dispositif de radiologie fournissant des images de haute résolution d'une partie quelconque désirée d'un sujet, et d'autre part de mettre en oeuvre un tel dispositif selon un procédé avantageux.

Un autre but de l'invention est de permettre de réaliser un dispositif de radiologie en temps réel dans lequel entre autres les images ne sont quasiment pas déformées.

Afin d'atteindre ces buts, l'invention propose selon un premier aspect un dispositif de radiologie selon la revendication 1.

Des aspects préférés mais non limitatifs du dispositif selon l'invention sont repris dans les revendications 2 a 15.

Selon un deuxième aspect, l'invention porte également sur l'utilisation d'une des formes de réalisation du dispositif décrites ci-dessus, pour des examens radiologiques en temps réel (notamment pour des applications dans les domaines industriel et maritime).

D'autres caractéristiques, buts et avantages de l'invention apparaîtront mieux à la lecture de la description suivante de trois formes de réalisation de l'invention, faite en référence aux dessins annexés, sur lesquels :
- les figures 1 à 3 sont des représentations du type bloc-diagramme de trois formes de réalisation d'un dispositif de radiologie selon l'invention,
- la figure 4 est une représentation schématique d'éléments d'acquisition d'images pouvant être mis en oeuvre dans un dispositif de radiologie selon l'invention.

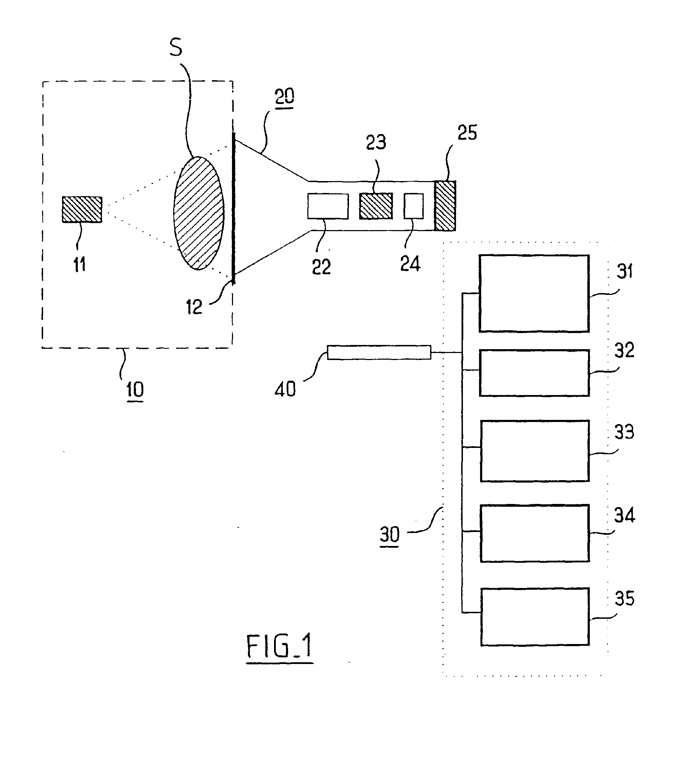

En référence tout d'abord à la figure 1, on a représenté schématiquement un premier module 10 comprenant une source de rayons X 11, et un écran fluorescent 12. Ce module 10 peut être un module de fluoroscopie classique, l'écran 12 délivrant en sortie du module 10 une image visible primaire correspondant à la trace des rayons X émis par la source 11 après leur traversée d'un sujet S qui est intercalé entre la source 11 et l'écran 12.

Le dispositif comprend également un deuxième module, référencé 20, d'acquisition des images primaires et de formation d'images secondaires. Comme on va le voir plus en détail dans la suite de ce texte, la couverture spatiale de ces images secondaires peut correspondre à celle des images primaires formées sur l'écran fluorescent 12, ou bien à une partie seulement de ces images primaires.

Le module 20 comprend dans une enceinte étanche à la lumière :
- un ensemble optique 22 d'agrandissement variable d'images, focalisé sur l'écran fluorescent 12,
- un ensemble 23 intensificateur d'images pour produire à partir des images agrandies par l'ensemble 22 des images d'intensité lumineuse supérieure,
- une lentille 24 de refocalisation pour reformer des images intensifiées en sortie de l'ensemble 23,
- un capteur optique 25, sur lequel la lentille 24 est focalisée, pour recueillir l'image agrandie et intensifiée et la convertir en une images secondaire analogique discrétisée. Ce capteur peut être une matrice de type CCD par exemple.

Les éléments optiques 22, 23, 24 et 25 du module 20, qui sont assemblés en série et forment ainsi une chaîne optique, sont en outre montés sur un système de déplacement deux axes, non représenté sur les figures. Ce système peut déplacer ces éléments optiques dans les deux directions coplanaires à l'écran fluorescent 12, afin d'amener en particulier l'ensemble d'agrandissement 22 en regard d'une zone désirée quelconque de cet écran.

Le dispositif comprend un troisième module 30 de traitement et de distribution des images issues du module 20. Ce module 30, qui constitue une unité centrale de commande du dispositif, comprend dans la forme de réalisation représentée sur la figure 1 les éléments suivants qui sont reliés entre eux :
- une unité 31 comprenant des moyens électroniques pour numériser des signaux analogiques délivrés par le capteur 25, et pour traiter ces signaux,
- une unité 32 de stockage local d'images numériques,
- une unité 33 d'interface pour la communication de signaux vidéo (en provenance du capteur 25 et/ou à destination de periphériques vidéo externes),
- une unité 34 d'interface pour la communication de signaux numériques,
- et une unité de commande 35 d'exposition et d'agrandissement.

Le module 30 comprend également des moyens (non représentés sur la figure) de commande du système de déplacement des éléments de la chaîne optique du module 20, et en particulier de l'ensemble d'agrandissement 22.

Le dispositif comprend enfin une interface 40 de visualisation locale d'images pouvant consister en un écran vidéo haute résolution, raccordé aux unités du module 30. Cette interface 40 peut être un écran numérique recevant les images secondaires numérisées par l'unité 31 du module 30.

Ce dispositif peut fonctionner selon un mode continu, également objet de l'invention, décrit ci-dessous :

Le sujet étant exposé aux radiations de la source 11 pour former une image dynamique sur l'écran 12, le radiologue peut visualiser en temps réel sur l'écran 40 une image secondaire correspondant à la totalité de l'image primaire formée sur l'écran 12, couvrant par exemple un champ élargi du sujet à l'intérieur duquel le radiologue recherche des zones spécifiques d'étude.

Grâce aux moyens de commande du système de déplacement du module 30 et à l'unité 35 de commande d'exposition et d'agrandissement, le radiologue peut ensuite commander le déplacement continu de la chaîne optique formée par les éléments 22, 23, 24 et 25 dans un plan parallèle au plan de l'écran 12, ainsi que le degré d'agrandissement de l'image formée en sortie de la lentille 22 et transmise aux autres éléments optiques de la chaîne. Ce degré d'agrandissement peut être fixé par le radiologue à toute valeur désirée dans une plage donnée, qui dépend du choix de l'ensemble 22.

On notera que tout ou partie des éléments du module 30 peuvent être situés à distance des autres constituants du dispositif (en particulier des modules 10 et 20), par exemple dans une pièce séparée dédiée à la commande du dispositif et à la visualisation des images, ou encore dans un bâtiment séparé. Dans ce cas, la longueur de la liaison entre le module 30 et le module 20 (qui est constituée d'au moins un câble pour transmettre les images du capteur optique 25 au module 30, et pour transmettre aux éléments de la chaîne optique du module 20 les commandes issues du module 30) est adaptée.

Pour commander ces moyens de déplacement, d'exposition et d'agrandissement, le radiologue dispose d'une interface (non représentée) qui peut être associée à l'écran 40 de visualisation. Cette interface peut utiliser un câble de commande, relié au module 30 pour activer manuellement le processus de sélection et de capture d'images.

L'écran 40 peut être de tout type connu, entre autres un écran à cristaux liquides. Le module 30 peut également être associé à un ordinateur de commande de type PC auquel sont rapportées les commandes du dispositif (commandes de l'exposition, du déplacement de la chaîne optique et de l'agrandissement, ...). Un tel PC peut contenir un programme d'utilisation du dispositif, mettant en oeuvre un menu pour la commande du dispositif s'affichant sur l'écran 40 en combinaison avec les images de sortie du dispositif.

Il est important de remarquer ici que selon l'invention, on n'altère aucunement la résolution de l'image en modifiant le degré d'agrandissement, étant donné que l'ensemble 22 qui est uniquement composé d'éléments optiques ne procède aucunement à une discrétisation de l'image.

Il est ainsi possible au radiologue d'identifier à partir d'images ayant un champ élargi des zones d'intérêt spécifique, puis de déplacer les éléments optiques et de zoomer sur la ou les zone(s) choisie(s), tout en obtenant en sortie de l'ensemble 22 une image dont la résolution n'est aucunement altérée.

On remarquera que selon l'invention, l'enchaînement des prises de vues de différentes zones avec différents degrés d'agrandissement est réalisé de manière continue, ce qui est un avantage en termes de facilité d'utilisation.

L'image (agrandie ou non) est transmise par l'ensemble 22 à l'intensificateur optique 23, qui sera de préférence constituée à partir d'éléments dits "channel multiplyer plate (ou MCP selon l'acronyme en usage)" selon la terminologie anglo-saxonne. On trouvera un exemple de réalisation d'un tel élément dans le brevet US 3 660 668. Il est également possible de relier en série plusieurs éléments intensificateurs du type MCP, et d'obtenir ainsi un gain optique de l'ordre de 10³ à 10⁷.

Grâce a l'intensificateur optique 23, l'intensité du rayonnement de la source de rayons X 11 est limite a un niveau équivalent a celui classiquement mis en oeuvre dans les dispositifs de fluoroscopie à intensificateurs, c'est a dire que l'exposition prolongée du radiologue et du sujet au rayonnement ne présente pas un risque sanitaire.

On remarquera en outre qu'un intensificateur optique construit à partir d'éléments du type MCP ne comporte pas d'éléments de géométrie bombée tels que l'écran d'entrée des intensificateurs d'images employé classiquement les dispositifs de fluoroscopie.

Ceci constitue un avantage dans la mesure où cette caractéristique n'introduit pas de distorsion de l'image. L'ensemble d'agrandissement 22 est ainsi le seul élément optique du dispositif comprenant des parties courbes, de sorte que les aberrations et déformations optiques de certaines zones de l'image sont réduites au minimum.

Le radiologue peut ainsi procéder à un examen continu de différentes zones du sujet, en déplaçant la chaîne optique formée par les éléments 22, 23, 24 et 25 et en commandant par l'intermédiaire de l'unité 35 l'agrandissement de l'image sur chaque zone désirée successivement, l'agrandissement de l'image n'altérant pas la résolution spatiale de l'image.

On notera que l'agrandissement de l'image est en outre ajustable de manière continue par l'intermédiaire de l'unité de commande 35, ce qui augmente encore la souplesse d'utilisation du dispositif selon l'invention.

Il est également possible d'intégrer au module 30 une unité de programmation de déplacements et d'agrandissements successifs, afin de réaliser un programme d'examens prédéterminés.

Il est également possible de désolidariser physiquement l'ensemble 22 des autres éléments 23. 24. 25 de la chaîne optique du module 20, et de commander seulement le déplacement de cet ensemble 22 en regard de l'écran fluorescent 12, des moyens de transmission d'images tels qu'une liaison par fibres optiques étant alors prévus entre l'ensemble 22 et l'intensificateur 23.

Il est également à noter que le processus d'examen radiologique décrit ci-dessus peut se dérouler sans changer l'intensité du rayonnement X auquel est exposé le sujet. En effet, l'agrandissement de l'image n'altérant pas sa résolution, il n'est pas nécessaire d'augmenter la dose de rayonnement pour visualiser une zone de détail de dimension réduite.

Les images numériques peuvent être stockées par l'unité 32 et distribuées à tout type de périphérique numérique (ou analogique grâce à l'unité 33 d'interface de communication de signaux vidéo). Ces périphériques peuvent être des écrans de visualisation, des imprimantes haute résolution, des moyens de stockage déportés et d'archivage, etc. Ils peuvent être localisés sur le même site que le dispositif décrit plus haut, ou être déportés sur d'autres sites munis d'une liaison avec le module 30.

Grâce au dispositif décrit ci-dessus, le radiologue peut également effectuer une première prise de vue rapide d'un champ élargi contenant la totalité du sujet, puis étudier à loisir l'image produite, la source 11 étant inactivée. Après avoir identifié les zones spécifiques qu'il désire étudier plus en détail, le radiologue peut alors réactiver la source 11 pour obtenir des images détaillées de ces zones avec la pleine résolution de l'ensemble de capteurs 25. Ce mode d'utilisation de l'invention permet de réduire encore le niveau d'exposition du radiologue et du sujet au rayonnement.

Le module 30 peut également comprendre, notamment dans l'unité 31, tous les moyens connus de traitement numérique de l'image, tels que des moyens de manipulation de l'image (choix de zones, rotation, traitement du contraste et opérations de seuillage, ...).

On notera que la résolution des images produites par le dispositif selon l'invention est très nettement supérieure à celle des images produites par le dispositif de fluoroscopie mettant en oeuvre des intensificateurs d'images. La résolution des images de ces dispositifs est en effet limitée par la résolution des intensificateurs, qui est au mieux de l'ordre de une à deux paires de lignes par millimètre ; l'utilisation d'intensificateurs du type MCP, combinée avec l'absence de discrétisation des images lors de leur agrandissement, permet d'atteindre une résolution supérieure.

On notera en outre que grâce aux moyens de traitement et de distribution de l'image numérique du module 30, le dispositif selon l'invention offre de multiples possibilités d'exploitation pratique. Les fichiers des images peuvent en effet être facilement transmis par des moyens électroniques à d'autres sites afin, par exemple, de solliciter des avis d'experts différents.

On remarquera également que l'utilisation d'imprimantes, ou de tout autre type de périphérique connu pour éditer et/ou imprimer sur un support tel que du papier (classique ou de qualité photo selon les besoins) les images numériques issues du module 30, constitue un moyen extrêmement souple et économique d'obtenir des clichés équivalents à des clichés radiographiques, de sorte que le dispositif selon l'invention peut être utilisé comme appareil de radiographie, ou de fluoroscopie.

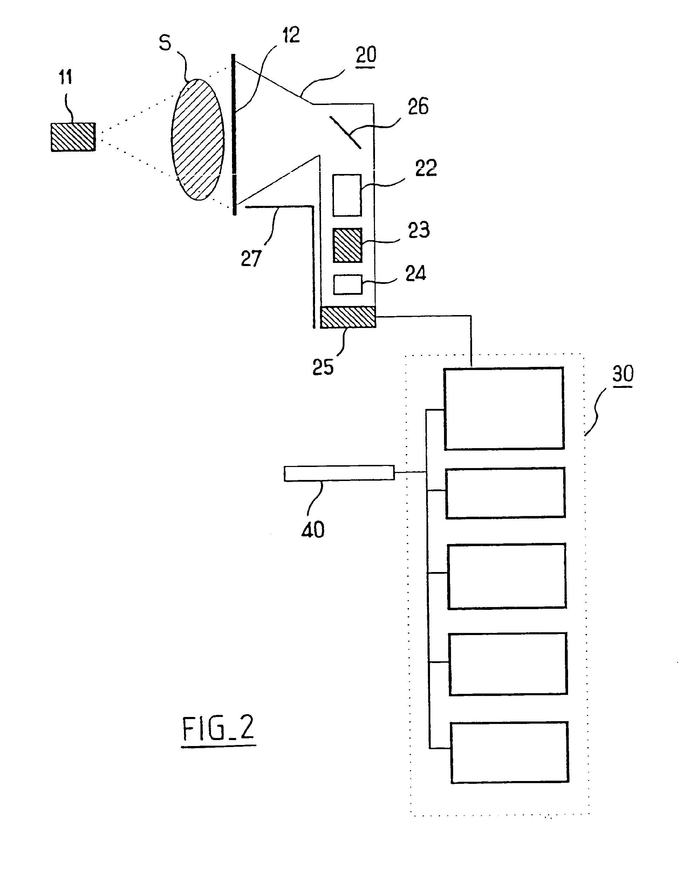

La figure 2 représente une deuxième forme de réalisation du dispositif selon l'invention, dans laquelle le module 20 est coudé à 90°, l'image de l'écran fluorescent 12 étant renvoyée vers la chaîne optique de la lentille 22 par l'intermédiaire d'un miroir de renvoi 26.

Dans cette forme de réalisation, la chaîne optique du module 20 est orientée généralement parallèlement au plan de l'écran 12, un miroir 26 de renvoi à 90° renvoyant les images de l'écran 12 sur l'ensemble d'agrandissement 22. Il est également prévu un blindage 27 en forme de « T » afin de protéger les éléments de la chaîne optique du rayonnement X.

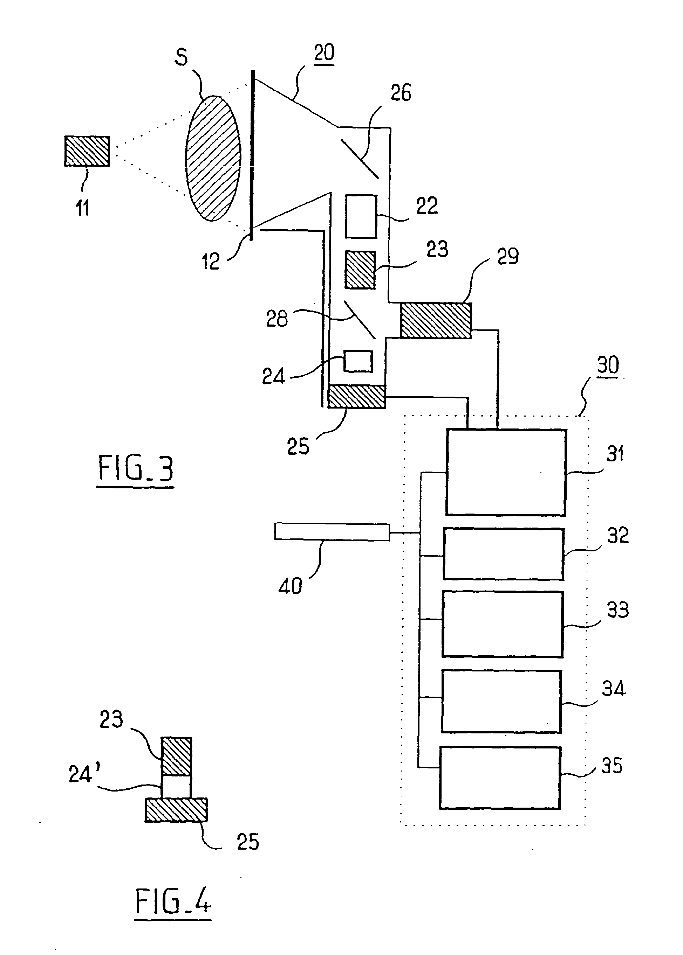

La figure 3 présente une troisième forme de réalisation de l'invention dans laquelle on a en outre intercalé entre l'intensificateur d'images et la lentille 24 de refocalisation un miroir prismatique 28 de séparation de l'image, afin de séparer les images transmises par l'intensificateur optique en deux faisceaux dirigés respectivement vers le capteur 25 et vers une caméra vidéo numérique 29, ces deux éléments étant reliés à l'unité 31 du module 30.

Cette troisième forme de réalisation permet l'acquisition séparée d'images dynamiques (par la caméra 29) et d'images statiques (par le capteur 25) ; cette disposition permet d'augmenter encore la souplesse et les performances du dispositif.

La figure 4 représente une variante de réalisation d'éléments de la chaîne optique du dispositif selon l'invention, dans laquelle l'intensificateur d'images 23 est relié au capteur 25 par un réseau de fibres optiques 24' en remplacement de la lentille de refocalisation 24.

Bien entendu, le dispositif selon l'invention n'est pas limité aux formes de réalisation décrites ci-dessus, mais peut être réalisé selon toute variante à la portée de l'homme du métier.

L'utilisation d'un tel dispositif n'est pas non plus limitée au domaine médical ; le dispositif décrit ci-dessus peut en effet également être mis en oeuvre dans tout autre domaine d'application des appareils de radiographie, et d'examen par rayons X en général.

En particulier, l'utilisation d'un tel dispositif pour l'inspection ou l'analyse qualitative non destructive de matériaux, par exemple dans le domaine industriel (inspection de parois ou de canalisations, ...), maritime (inspection de navires ou de sous-marins, ...), etc, permet d'accéder aux avantages de souplesse d'utilisation en temps réel décrits ci-dessus à propos d'un examen médical..

## Revendications

1. Dispositif de radiologie comprenant une source (11) de rayons X pour exposer un sujet (S) au rayonnement de ladite source, des moyens (12) de conversion des rayons X en images optiques pour former des images optiques primaires, des moyens (20) pour transformer les images optiques primaires en des images optiques secondaires, et des moyens (40) pour présenter les images secondaires à un utilisateur, **caractérisé en ce que** les moyens pour former les images optiques secondaires comprennent une chaîne optique comprenant en succession, de la sortie du convertisseur vers la sortie du dispositif, un ensemble (22) d'agrandissement des images exposé directement aux images primaires desdits moyens de conversion (12), un ensemble (23) d'intensification optique des images agrandies et un capteur matriciel photosensible (25) pour constituer lesdites images secondaires, et **en ce que** l'ensemble (22) d'agrandissement est constitué uniquement d'éléments optiques n'effectuant pas de discrétisation des images.

2. Dispositif de radiologie selon la revendication 1, **caractérisé en ce que** l'ensemble (22) d'agrandissement est un ensemble (22) d'agrandissement variable, apte à agrandir les images selon un coefficient d'agrandissement désiré à l'intérieur d'une plage donnée.

3. Dispositif de radiologie selon l'une des revendications précédentes, **caractérisé en ce qu'**il comprend des moyens de déplacement des éléments de la chaîne optique dans un plan généralement parallèle au plan moyen des moyens de conversion.

4. Dispositif de radiologie selon la revendication précédente, **caractérisé en ce qu'**il comprend une unité centrale de commande (30) pour commander le déplacement des éléments de la chaîne optique.

5. Dispositif de radiologie selon la revendication précédente, **caractérisé en ce que** l'unité centrale de commande est physiquement éloignée des autres éléments du dispositif.

6. Dispositif de radiologie selon l'une des revendications précédentes, **caractérisé en ce qu'**il comprend des moyens de contrôle de l'exposition et du degré d'agrandissement des images.

7. Dispositif de radiologie selon l'une des revendications précédentes, **caractérisé en ce que** l'ensemble (23) d'intensification optique des images comprend des composants du type MCP.

8. Dispositif de radiologie selon l'une des revendications précédentes, **caractérisé en ce qu'**il comprend des moyens (31) de numérisation des images secondaires issues du capteur matriciel photosensible.

9. Dispositif de radiologie selon la revendication précédente, **caractérisé en ce qu'**il comprend des interfaces de distribution des images à destination de périphériques numériques.

10. Dispositif de radiologie selon l'une des revendications précédentes, **caractérisé en ce qu'**il comprend un écran de visualisation des images secondaires numérisées.

11. Dispositif de radiologie selon l'une des revendications précédentes, **caractérisé en ce que** les moyens (12) de conversion des rayons X en images optiques consistent en un écran de fluoroscopie du type écran à revêtement de phosphore.

12. Dispositif de radiologie selon l'une des revendications précédentes, **caractérisé en ce que** ladite chaîne optique est dirigée selon un axe différent de la normale au plan moyen des moyens (12) de conversion des rayons X en images optiques, le dispositif comprend un miroir de renvoi des images primaires vers la chaîne optique et le dispositif comprend un blindage (27) pour protéger les éléments de la chaîne optique des rayons X.

13. Dispositif de radiologie selon l'une des revendications précédentes, **caractérisé en ce que** la chaîne optique comprend une lentille de refocalisation (24).

14. Dispositif de radiologie selon l'une des revendications précédentes, **caractérisé en ce qu'**il comprend un miroir (28) de séparation des images issues de l'ensemble (23) d'intensification et une caméra vidéo numérique (29).

15. Dispositif de radiologie selon l'une des revendications précédentes. **caractérisé en ce que** le couplage optique entre l'ensemble (23) d'intensification et le capteur (25) est assuré par des fibres optiques (24').

16. Utilisation d'un dispositif de radiologie selon l'une des revendications précédentes pour examen médical en temps réel.

17. Utilisation d'un dispositif de radiologie selon l'une des revendications 1 à 15 pour inspection qualitative non destructive de matériaux, en particulier dans le domaine industriel ou maritime.

## Claims

1. A radiology device comprising an X-ray source (11) for exposing a subject (S) to the radiation of said source, means (12) for converting the X-rays into optical images so as to form primary optical images, means (20) for transforming the primary optical images into secondary optical images, and means (40) for displaying the secondary images to a user, **characterized in that** the means for forming the secondary optical images comprise an optical chain comprising in succession, from the output of the converter to the output of the device, an image enlargement assembly (22) exposed directly to the primary images from said conversion means (12), an assembly (23) for optical intensification of the enlarged images and a photosensitive matrix sensor (25) for making said secondary images, and **in that** the enlargement assembly (22) is made up solely of optical elements performing no discretization of the images.

2. The radiology device as claimed in claim 1, **characterized in that** the enlargement assembly (22) is a variable enlargement assembly (22), able to enlarge the images according a desired enlargement coefficient within a given range.

3. The radiology device as claimed in one of the preceding claims, **characterized in that** it comprises means for moving the elements of the optical chain in a plane generally parallel to the midplane of the conversion means.

4. The radiology device as claimed in the preceding claim, **characterized in that** it comprises a central control unit (30) for controlling the movement of the elements of the optical chain.

5. The radiology device as claimed in the preceding claim, **characterized in that** the central control unit is physically distanced from the other elements of the device.

6. The radiology, device as claimed in one of the preceding claims, **characterized in that** it comprises means of monitoring the exposure and the degree of enlargement of the images.

7. The radiology device as claimed in one of the preceding claims, **characterized in that** the assembly (23) for optical intensification of the images comprises components of the MCP type.

8. The radiology device as claimed in one of the preceding claims, **characterized in that** it comprises means (31) for digitizing the secondary images arising from the photosensitive matrix sensor.

9. The radiology device as claimed in the preceding claim, **characterized in that** it comprises interfaces for distributing the images destined for digital peripherals.

10. The radiology device as claimed in one of the preceding claims, **characterized in that** it comprises a screen for visualizing the digitized secondary images.

11. The radiology device as claimed in one of the preceding claims, **characterized in that** the means (12) for converting the X-rays into optical images consist of a fluoroscopy screen of the phosphor coating screen type.

12. The radiology device as claimed in one of the preceding claims, **characterized in that** said optical chain is directed along an axis different from the normal to the midplane of the means (12) for converting the X-rays into optical images, the device comprises a mirror for deflecting the primary images to the optical chain and the device comprises a shield (27) for protecting the elements of the optical chain from the X-rays.

13. The radiology device as claimed in one of the preceding claims, **characterized in that** the optical chain comprises a refocusing lens (24).

14. The radiology device as claimed in one of the preceding claims, **characterized in that** it comprises a mirror (28) for separating the images arising from the intensification assembly (23) and a digital video camera (29).

15. The radiology device as claimed in one of the preceding claims, **characterized in that** the optical coupling between the intensification assembly (23) and the sensor (25) is effected by optical fibers (24').

16. The use of a radiology device as claimed in one of the preceding claims for real-time medical examination.

17. The use of a radiology device as claimed in one of claims 1 to 15 for nondestructive qualitative inspection of materials, in particular in the industrial or maritime sector.

## Patentansprüche

1. Radiologische Vorrichtung, umfassend eine Röntgenstrahlen-Quelle (11), um einen Gegenstand (S) der Strahlung der besagten Quelle auszusetzen, Mittel (12) zur Umwandlung der Röntgenstrahlen in optische Bilder, um primäre optische Bilder zu bilden, Mittel (20), um die primären optischen Bilder in sekundäre optische Bilder umzuwandeln, und Mittel (40), um einem Anwender die sekundären Bilder darzustellen, **dadurch gekennzeichnet, dass** die Mittel zur Bildung der sekundären optischen Bilder eine optische Kette umfassen, die vom Ausgang des Umwandlers zum Ausgang der Vorrichtung aufeinander folgend eine Anordnung zur Bildvergrößerung (22) umfasst, welche direkt auf die primären Bilder besagter Umwandlungsmittel (12) ausgeführt wird, eine Anordnung zur optischen Verstärkung (23) der vergrößerten Bilder und einen lichtempfindlichen Matrizensensor (25), um die besagten sekundären Bilder zu bilden, und dass die Anordnung zur Bildvergrößerung (22) nur aus optischen Elementen besteht, die keine Diskretisierung der Bilder durchführen.

2. Radiologische Vorrichtung gemäß Anspruch 1, **dadurch gekennzeichnet, dass** die Anordnung zur Bildvergrößerung (22) eine variable Anordnung zur Bildvergrößerung ist (22), geeignet, die Bilder innerhalb eines vorgegebenen Bereichs gemäß eines gewünschten Vergrößerungskoeffizienten zu vergrößern.

3. Radiologische Vorrichtung gemäß einem der vorhergehenden Ansprüche, **dadurch gekennzeichnet, dass** sie Mittel zur Verschiebung der Elemente der optischen Kette in einer Ebene, im Allgemeinen parallel zur mittleren Ebene der Umwandlungsmittel, umfasst.

4. Radiologische Vorrichtung gemäß dem vorhergehenden Anspruch, **dadurch gekennzeichnet, dass** sie eine zentrale Steuerungseinheit (30) umfasst, um die Verschiebung der Elemente der optischen Kette zu steuern.

5. Radiologische Vorrichtung gemäß dem vorhergehenden Anspruch, **dadurch gekennzeichnet, dass** die zentrale Steuerungseinheit physisch von den anderen Elementen der Vorrichtung entfernt ist.

6. Radiologische Vorrichtung gemäß einem der vorhergehenden Ansprüche, **dadurch gekennzeichnet, dass** sie Mittel zur Kontrolle der Belichtung und des Vergrößerungsgrades der Bilder umfasst.

7. Radiologische Vorrichtung gemäß einem der vorhergehenden Ansprüche, **dadurch gekennzeichnet, dass** die Anordnung (23) zur optischen Verstärkung der Bilder Komponenten vom Typ MCP umfasst.

8. Radiologische Vorrichtung gemäß einem der vorhergehenden Ansprüche, **dadurch gekennzeichnet, dass** sie Mittel zur Digitalisierung (31) der von dem lichtempfindlichen Matrizensensor stammenden sekundären Bilder umfasst.

9. Radiologische Vorrichtung gemäß dem vorhergehenden Anspruch, **dadurch gekennzeichnet, dass** sie Schnittstellen zur Verteilung der Bilder an digitalen Peripheriegeräten umfasst.

10. Radiologische Vorrichtung gemäß einem der vorhergehenden Ansprüche, **dadurch gekennzeichnet, dass** sie einen Bildschirm zur Visualisierung der digitalisierten sekundären Bilder umfasst.

11. Radiologische Vorrichtung gemäß einem der vorhergehenden Ansprüche, **dadurch gekennzeichnet, dass** die Mittel (12) zur Umwandlung der Röntgenstrahlen in optische Bilder aus einem Fluoroskopie-Bildschirm vom Typ eines Bildschirms mit Phosphor-Verkleidung bestehen.

12. Radiologische Vorrichtung gemäß einem der vorhergehenden Ansprüche, **dadurch gekennzeichnet, dass** die besagte optische Kette entlang einer Achse, die verschieden ist von der Normalen zur mittleren Ebene der Mittel (12) zur Umwandlung der Röntgenstrahlen in optische Bilder, gelenkt wird, die Vorrichtung einen Spiegel zur Wiedergabe der primären Bilder in Richtung der optischen Kette umfasst und die Vorrichtung eine Abschirmung (27) umfasst, um die Elemente der optischen Kette vor Röntgenstrahlen zu schützen.

13. Radiologische Vorrichtung gemäß einem der vorhergehenden Ansprüche, **dadurch gekennzeichnet, dass** die optische Kette eine Linse zur Refokussierung (24) umfasst.

14. Radiologische Vorrichtung gemäß einem der vorhergehenden Ansprüche, **dadurch gekennzeichnet, dass** sie einen Spiegel (28) zur Trennung der aus der Anordnung zur Verstärkung (23) hervorgegangenen Bilder und eine digitale Video-Kamera (29) umfasst.

15. Radiologische Vorrichtung gemäß einem der vorhergehenden Ansprüche, **dadurch gekennzeichnet, dass** die optische Kopplung zwischen der Anordnung zur Verstärkung (23) und dem Sensor (25) durch optische Fasern (24) bereitgestellt wird.

16. Nutzung einer radiologischen Vorrichtung gemäß einem der vorhergehenden Ansprüche zur medizinischen Untersuchung in Echtzeit.

17. Nutzung einer radiologischen Vorrichtung gemäß einem der Ansprüche 1 bis 15 zur qualitativen, zerstörungsfreien Materialprüfung, insbesondere im industriellen oder maritimen Bereich.
